# EUROPÄISCHE PATENTSCHRIFT

(11) **EP 1 923 384 B1**
(45) Veröffentlichungstag und Bekanntmachung des Hinweises auf die Patenterteilung: **26.06.2013**
(21) Anmeldenummer: 07119408.8
(22) Anmeldetag: 26.10.2007
(51) Int. Cl.: C07C 319/08, C07C 319/02, C07C 323/12

(54) **Verfahren zur Herstellung von 2-Mercaptoethanol**
Method for manufacturing 2-mercapto ethanol
Procédé de fabrication de 2-mercaptoéthanol

(30) Priorität: 09.11.2006 EP 06123754
(43) Veröffentlichungstag der Anmeldung: 21.05.2008
(73) Patentinhaber: BASF SE, 67056 Ludwigshafen (DE)
(72) Erfinder: Rehfinger, Alwin, 67112, Mutterstadt (DE); Böhling, Ralf, 64653, Lorsch (DE); Schimpf, Axel, 69121, Heidelberg (DE); Mrzena, Frank, 67112, Mutterstadt (DE); Sager, Wilfried, 67112, Mutterstadt (DE)

(56) Entgegenhaltungen:
- GB-A- 1 296 452

## Beschreibung

Zur Darstellung der Erfindungs-Gattung ist im Oberbegriff des Anspruchs 1 von einem bekannten Verfahren im Sinne der CH-PS 533 092 ausgegangen, nach dem bisher 2-Mercaptoethanol durch Behandeln von Ethylenoxid mit Schwefelwasserstoff in Gegenwart eines Katalysators bzw. Lösungsmittels hergestellt wurde. Die Synthese wird in homogener flüssiger Phase bei höheren Temperaturen und unter erhöhtem Druck durchgeführt, wobei ein Gemisch aus Schwefelwasserstoff und Katalysator sowie das Ethylenoxid vorgewärmt und getrennt voneinander in eine rohrförmige Reaktionszone eingespeist werden. In der Reaktionszone werden Druck und Temperatur so aufeinander abgestimmt, dass sich keine Gasphase ausbildet. Auf diese Weise lässt sich, bezogen auf umgesetztes Ethylenoxid, eine Ausbeute an 2-Mercaptoethanol von 90% erreichen.

Zur Erzielung einer höheren Ausbeute an 2-Mercaptoethanol ist es aus der DE 31 22 285 A1 bekannt, Schwefelwasserstoff in gasförmigem Zustand mit flüssigem Ethylenoxid in Berührung zu bringen und den Druck dabei so einzustellen, dass sich die maximale Menge des Schwefelwasserstoffs in dem Ethylenoxid auflöst. Nachteilig ist indes die lange Reaktionszeit, die wiederum eine entsprechend groß dimensionierte Reaktionszone erfordert. Damit liegen auch die Reaktorkosten und die sicherheitsrelevante Vorratshaltung von Ethylenoxid und Schwefelwasserstoff entsprechend hoch.

Mit der Erfindung soll ein Verfahren zur Herstellung von 2-Mercaptoethanol bereitgestellt werden, mit dem sich das Potential möglichst hoher Reaktionsgeschwindigkeiten bei Aufrechterhaltung einer hochselektiven Umsetzung von Ethylenoxid mit Schwefelwasserstoff mit minimalen sicherheitstechnischen Anforderungen realisieren lässt.

Diese Aufgabe wird durch die im Kennzeichen des Anspruchs 1 enthaltenen Maßnahmen gelöst.

Zweckmäßige Weiterbildungen des erfindungsgemäßen Verfahrens ergeben sich aus den Unteransprüchen 2 bis 6.

Nach dem neuen Verfahren werden Ethylenoxid und Schwefelwasserstoff in Gegenwart eines Katalysators und/oder Lösungsmittels in einer mikrostrukturierten Reaktionszone umgesetzt. Unter einer mikrostrukturierten Reaktionszone im Sinne der Erfindung werden Zonen eines Reaktors mit parallelen Kanälen mit hydraulischen Kanaldurchmessungen von weniger als 2 mm, insbesondere weniger als 1 mm und einer spezifischen inneren Oberfläche von insgesamt mehr als 2000, vorzugsweise mehr als 4000 m²/m³ verstanden. Eine solche Reaktionszone wird im Allgemeinen aus modular zusammengesetzten Bauteilen gebildet, wobei die einzelnen Bauteile an jeweils übereinstimmenden Stellen Bohrungen oder Vertiefungen haben, die in Gebrauchsstellung Kanäle für die Stoff-, Reaktions- und Wärmeführung bilden. Mikrostrukturierte Reaktoren zeichnen sich durch eine sehr gute Wärmeabfuhr verbunden mit einem sehr schnellen Stofftransport aus.

Erfindungsgemäß werden das Ethylenoxid, der Schwefelwasserstoff und der Katalysator bzw. das Lösungsmittel miteinander vermischt und das Komponentengemisch in homogener flüssiger Phase in die Reaktionszone gefördert. Die homogene Verteilung der Einsatzstoffe erfolgt beispielsweise in einem feststehenden Mischbehälter mit einem zentralen blatt- oder scheibenförmigen Rührwerkzeug, in einer Mischpumpe oder einem Statikmischer bei einer Temperatur unterhalb der Reaktionstemperatur und einem Druck, der höher ist als der Dampfdruck des Schwefelwasserstoffs, so dass sich während des Zulaufs des Komponentengemischs in die Reaktionszone keine Gasphase ausbildet. Das vorgeschaltete Mischen der Komponenten wird durch Abstimmung von Temperatur, Verweilzeit und Wärmeaustauschfläche so durchgeführt, dass ein unkontrolliertes Reagieren des Gemischs, was unerwünscht ist, nicht eintritt.

Bei zweistufiger Vermischung der Komponenten wird zunächst ein Gemisch aus Katalysator bzw. Lösungsmittel und Schwefelwasserstoff hergestellt und anschließend Ethylenoxid zugemischt.

Das Molverhältnis von Ethylenoxid und Schwefelwasserstoff beträgt etwa 1:1 bis 1:2.

Als Katalysator bzw. Lösungsmittel wird Thiodiglykol verwendet. Vorteilhaft werden Ethylenoxid und Thiodiglykol im Molverhältnis von 1:0,001 bis 1:10 eingesetzt. Ein hoher Anteil an Thiodiglykol ist unkritisch. Es ist auch möglich, Thiodiglykol zusätzlich als zweiten flüssigen Strom in die Reaktionszone einzubringen.

Nach einer bevorzugten Ausführungsform wird das während des Verfahrens als Nebenprodukt gebildete Thiodiglykol nach partieller Abtrennung des 2-Mercaptoethanols ohne weitere Reinigung als Katalysator und/oder Lösungsmittel wiederverwendet. Das Thiodiglykol kann als Rückführstrom aus der nachgeschalteten Aufarbeitung direkt wieder der Reaktionszone zugeführt werden.

Erfindungsgemäß wird das Komponentengemisch in homogener flüssiger Phase unter einem Druck von 4 bis 250 bar, bevorzugt 20 bis 150 bar, besonders bevorzugt 90 bis 110 bar in die mikrostrukturierte Reaktionszone gefördert, in Teilströme aufgeteilt und auf Reaktionstemperatur erwärmt. Die Umsetzung des Ethylenoxids mit Schwefelwasserstoff erfolgt bei einer Temperatur von 40 bis 200°C, vorzugsweise 70 bis 180°C, insbesondere etwa 110°C innerhalb von 0,1 bis 15 min., zweckmäßig innerhalb von 0,5 bis 1,5 min. Auf diese Weise ist eine Raumzeitausbeute an 2-Mercaptoethanol von bis zu 30 t/hm³ erreichbar. Die Teilströme werden am Ende der Reaktionszone wieder zusammengeführt und es wird das Reaktionsprodukt, ausgetragen und durch fraktionierte Destillation aufgearbeitet.

Bei der Umsetzung kann es in der Reaktionszone zu einer begrenzten Ausbildung einer Schwefelwasserstoff-Gasphase kommen, die sich indes nicht nachteilig auswirkt. Vielmehr wird durch die dabei entstehende sogenannte Blasenkettenströmung die Wärmeauskopplung in dem Komponentengemisch verbessert sowie die Rückvermischung der Komponenten unterdrückt, die ansonsten bei laminarer Strömung selektivitäts- und reaktionsgeschwindigkeitsreduzierend wirkt. Bei zu starker Erwärmung des Komponentengemischs wird dessen Verweilzeit in der Reaktionszone entsprechend reduziert und der Umsatz sinkt ab.

Das Verfahren der Erfindung lässt sich mit besonderem Vorteil kontinuierlich durchführen, indem in einem Mischbehälter ununterbrochen Ethylenoxid, Schwefelwasserstoff und Katalysator bzw. Lösungsmittel miteinander vermischt und das Gemisch in flüssiger Phase in die mikrostrukturierte Reaktionszone gefördert wird, während gleichzeitig am Ende der Reaktionszone Reaktionsprodukt ausgetragen wird. Es ist aber auch möglich, das Komponentengemisch der Reaktionszone diskontinuierlich zuzuführen und das Reaktionsprodukt in kurzen Zeitabständen aus dieser zu entnehmen.

Gegenüber dem Stand der Technik können nach dem erfindungsgemäßen Verfahren hohe Raumzeitausbeuten sowie eine hohe Selektivität von bis zu 97% 2-Mercaptoethanol, bezogen auf umgesetztes Ethylenoxid, erzielt werden. Die mikrostrukturierte Reaktionszone ist störunanfällig und kann besonders kleinbauend ausgeführt sein. Zusätzliche Vorkehrungen für die gleichmäßige Aufteilung des Komponentengemischs in die einzelnen Kanäle sind nicht erforderlich.

2-Mercaptoethanol ist für zahlreiche Anwendungen geeignet, beispielsweise zur Herstellung von PVC-Stabilisatoren, als Regler für Polymerisationsreaktionen, als Prozesschemikalie bei photographischen Verfahren, als Synthesebaustein für die Herstellung von Agrochemikalien, als Saatbeizmittel und in der Lederindustrie.

Das Verfahren der Erfindung wird durch folgende Beispiele näher erläutert:

### Beispiel 1

In einem Rührbehälter mit einem Volumen von 20 ml wurden stündlich 31,6 g Ethylenoxid (EO), 32,8 g Schwefelwasserstoff (H₂S) und 39,3 g Thiodiglykol (TDG) bei Raumtemperatur und einem Druck von 92 bar miteinander vermischt und das homogenisierte Komponentengemisch kontinuierlich in eine mikrostrukturierte Reaktionszone bestehend aus vier parallelen und symmetrisch zueinander angeordneten Kanälen einer Länge von 1,3 m und einer lichten Weite von 0,8 mm eindosiert. Das Komponentengemisch wurde in den Kanälen mit Hilfe eines Thermostatbades erwärmt und unter einem Druck von 92 bar durch die Reaktionszone gefördert. Die Temperatur des Thermostatbades betrug 141°C. Die Temperatur in der im Thermostatbad eingetauchten Kanalwandung lag bereits nach einer Eintauchstrecke von 5 cm näher als 10 K an der umgebenden Badtemperatur. Die Verweilzeit des Komponentengemischs in der Reaktionszone 1,2 min. Am Ende der Reaktionszone wurde das Reaktionsprodukt auf 1 bar entspannt, auf 20°C abgekühlt und auf einen Dünnschichtverdampfer mit rotierenden Wischblättern aufgegeben. Bei ca. 40°C wurde in dem Dünnschichtverdampfer nicht umgesetztes EO und H₂S durch Strippung mit Stickstoff vollständig und ohne Verluste an Mercaptoethanol abgetrennt. In dieser Versuchseinstellung wurde Ethylenoxid zu 100 % umgesetzt. Der flüssige Austragsstrom des Dünnschichtverdampfers enthielt 54,2 g/h Mercaptoethanol. Dies entspricht einer Ausbeute bzw. Selektivität bezogen auf Ethylenoxid von 96,5 mol-%.

### Beispiel 2

Wie in Beispiel 1 beschrieben, wurden 107 g/h eines Komponentengemisch aus EO, H₂S und TDG mit einem Molverhältnis von 1 : 1,37 : 0,44 in einer miktrostrukturierten Reaktionszone mit 4 Kanälen einer Länge von 1,3 m und einer lichten Weite von 0,8 mm kontinuierlich bei einem Druck von 92 bar umgesetzt. Die Temperatur des Thermostatbades betrug 131°C. Die Verweilzeit des Komponentengemischs in der Reaktionszone lag bei 1,2 min. Ethylenoxid wurde zu 90,5 % umgesetzt. Die Selektivität zu Mercaptoethanol betrug wie im Beispiel 1 96,5 mol-% bezogen auf umgesetztes Ethylenoxid.

### Beispiel 3

Wie in Beispiel 1 beschrieben, wurden 103 g/h eines Komponentengemisch aus EO, H₂S und TDG mit einem Molverhältnis von 1 : 1,33 : 0,43 in einer mikrostrukturierten Reaktionszone mit 4 Kanälen einer Länge von 1,7 m und einer lichten Weite von 0,8 mm kontinuierlich bei einem Druck von 60 bar umgesetzt. Die Temperatur des Thermostatbades betrug 141°C. Ethylenoxid wurde zu 91,5 % umgesetzt. Die Selektivität zu Mercaptoethanol betrug wie im Beispiel 1 96,5 mol-% bezogen auf umgesetztes Ethylenoxid.

## Patentansprüche

1. Verfahren zur Herstellung von 2-Mercaptoethanol durch Umsetzung von Ethylenoxid mit Schwefelwasserstoff in Gegenwart eines Katalysators und/oder Lösungsmittels bei höheren Temperaturen und unter erhöhtem Druck, **dadurch gekennzeichnet, dass** die Umsetzung in einer mikrostrukturierten Reaktionszone mit parallelen Kanälen einer spezifischen inneren Oberfläche von insgesamt mehr als 2000 m²/m³ durchgeführt wird, wobei das Ethylenoxid, der Schwefelwasserstoff und der Katalysator bzw. das Lösungsmittel miteinander vermischt und in homogener flüssiger Phase unter einem Druck von 4 bis 250 bar in die Reaktionszone gefördert und bei einer Temperatur von 40 bis 200°C innerhalb von 0,1 bis 15 min. umgesetzt werden und das Reaktionsprodukt ausgetragen wird.

2. Verfahren nach Anspruch 1, **dadurch gekennzeichnet, dass** Ethylenoxid und Schwefelwasserstoff im Molverhältnis von etwa 1:1 bis 1:2 eingesetzt werden.

3. Verfahren nach Anspruch 1, **dadurch gekennzeichnet, dass** Ethylenoxid und Katalysator und/oder Lösungsmittel im Molverhältnis von 1:0,001 bis 1:10 eingesetzt werden.

4. Verfahren nach Anspruch 1, **dadurch gekennzeichnet, dass** als Katalysator und/oder Lösungsmittel Thiodiglykol eingesetzt wird.

5. Verfahren nach Anspruch 4, **dadurch gekennzeichnet, dass** das während des Verfahrens als Nebenprodukt gebildete Thiodiglykol nach partieller Abtrennung des 2-Mercaptoethanols ohne weitere Reinigung als Katalysator und/oder Lösungsmittel wiederverwendet wird.

6. Verfahren nach einem oder mehreren der Ansprüche 1 bis 5, **dadurch gekennzeichnet, dass** die Herstellung von 2-Mercaptoethanol kontinuierlich durchgeführt wird.

## Claims

1. A process for preparing 2-mercaptoethanol by reacting ethylene oxide with hydrogen sulfide in the presence of a catalyst and/or solvent at elevated temperatures and under superatmospheric pressure, wherein the reaction is carried out in a microstructured reaction zone having parallel channels having a total specific internal surface area of more than 2000 m²/m³, with the ethylene oxide, the hydrogen sulfide and the catalyst and/or the solvent being mixed with one another and conveyed as a homogeneous liquid phase under a pressure of from 4 to 250 bar into the reaction zone and reacted at a temperature of from 40 to 200°C within a period of from 0.1 to 15 minutes and the reaction product being discharged.

2. The process according to claim 1, wherein ethylene oxide and hydrogen sulfide are used in a molar ratio of from about 1:1 to 1:2.

3. The process according to claim 1, wherein ethylene oxide and catalyst and/or solvent are used in a molar ratio of from 1:0.001 to 1:10.

4. The process according to claim 1, wherein thiodiglycol is used as catalyst and/or solvent.

5. The process according to claim 4, wherein the thiodiglycol formed as by-product during the process is, after part of the 2-mercaptoethanol has been separated off, reused without further purification as catalyst and/or solvent.

6. The process according to one or more of claims 1 to 5, wherein the preparation of 2-mercaptoethanol is carried out continuously.

## Revendications

1. Procédé pour la production de 2-mercaptoéthanol par mise en réaction d'oxyde d'éthylène avec de l'hydrogène sulfuré en présence d'un catalyseur et/ou solvant à des températures relativement élevées et sous une pression élevée, **caractérisé en ce que** la réaction est effectuée dans une zone de réaction microstructurée à canaux parallèles ayant une surface spécifique interne d'au total plus de 2 000 m² /m³, l'oxyde d'éthylène, l'hydrogène sulfuré et le catalyseur ou le solvant étant mélangés entre eux et envoyés en phase liquide homogène, sous une pression de 4 à 250 bars, dans la zone de réaction et mis à réagir à une température de 40 à 200 °C en l'espace de 0,1 à 15 min. et le produit de réaction est déchargé.

2. Procédé selon la revendication 1, **caractérisé en ce qu'**on utilise l'oxyde d'éthylène et l'hydrogène sulfuré en un rapport molaire d'environ 1:1 à 1:2.

3. Procédé selon la revendication 1, **caractérisé en ce qu'**on utilise l'oxyde d'éthylène et le catalyseur et/ou le solvant en un rapport molaire de 1:0,001 à 1:10.

4. Procédé selon la revendication 1, **caractérisé en ce que** qu'on utilise comme catalyseur et/ou solvant le thiodiglycol.

5. Procédé selon la revendication 4, **caractérisé en ce que** le thiodiglycol formé en tant que produit secondaire au cours du processus est réutilisé, après séparation partielle du 2-mercaptoéthanol, sans être purifié davantage, en tant que catalyseur et/ou solvant.

6. Procédé selon une ou plusieurs des revendications 1 à 5, **caractérisé en ce que** la production de 2-mercaptoéthanol est effectuée en continu.
